Europäisches Patentamt

European Patent Office

Office européen des brevets

(19)

(11) Publication number:

**0 303 496**
**A1**

# (12) EUROPEAN PATENT APPLICATION

(21) Application number: **88307478.3**

(22) Date of filing: **12.08.88**

(51) Int. Cl.⁴: **A 61 L 31/00**
**A 61 L 27/00**

(30) Priority: **12.08.87 FR 8711485**

(43) Date of publication of application:
**15.02.89 Bulletin 89/07**

(84) Designated Contracting States: **DE GB**

(71) Applicant: **ETHICON INC.**
**U.S. Route 22**
**Somerville New Jersey 08876 (US)**

(72) Inventor: **Bilweis, Joseph,**
**10, L'Oree de Marly,**
**F-78560 Noisy le Roi (FR)**

(74) Representative: **Mercer, Christopher Paul et al**
**Carpmaels & Ransford 43, Bloomsbury Square**
**London WC1A 2RA (GB)**

(54) Resorbable surgical meshwork, prostheses of this meshwork and surgical uses thereof.

(57) The present invention relates to a resorbable surgical meshwork constituted by an assembly of at least two biocompatible resorbable threads of different nature of which one is based on regenerated oxidized cellulose, and the other one is a polyglycolic type synthetic material. It also relates to its surgical use.

The invention also extends to prostheses made from this meshwork and to its surgical use.

EP 0 303 496 A1

**Description**

# RESORBABLE SURGICAL MESHWORK, PROSTHESES OF THIS MESHWORK AND SURGICAL USES THEREOF

The present invention relates to a resorbable surgical meshwork, resorbable prostheses of this meshwork as well as use of the said meshwork or the said prostheses for repair of tissues and/or organs of a human or animal body.

The development of entirely resorbable materials has allowed surgical techniques to progress substantially.

A material is called "resorbable" or "absorbable" when, on introduction into a human or animal body, it is progressively metabolised by mechanisms such as hydrolysis or an enzymatic action and it thus becomes intimately combined with the living tissues. Such materials are biocompatible. Advantageously they permit avoidance of a second surgical operation which would be necessary for removing non-absorbable members arranged in a human or animal body during a first surgical repair operation.

Numerous meshworks of different resorbable materials have been proposed in the prior art. By "meshwork" is intended here a textile structure comparable to that of gauze or knitting, having interlaced threads.

Elemental meshworks are now known comprising a single type of thread: thus the applicant sells under the registered trade mark VICRYL® a meshwork of which the threads are constituted by a copolymer of glycolic acid and lactic acid. On account of its good mechanical strength, this meshwork gives entire satisfaction for supporting wounded organs and the regeneration of tissues; it is permeable by nature.

Elemental knitted fabrics sold under the registered trade mark SURGICEL® is also known. Made by knitting of threads of regenerated oxidized cellulose, they have good hemostatic properties but no particular mechanical strength.

Composite meshworks with several constituents have also been proposed, with the object of usefully combining diverse intrinsic properties of the constituents.

Thus the applicant has proposed a composite absorbable meshwork comprising a framework of synthetic threads of which one face is stratified with a layer of reconstituted collagen in order to provide the material obtained with an additional property of fluid-tightness, see French Patent Specification No. FR-A-2 577 807.

Such a material gives remarkable results where one wishes to associate fluid-tightness, good tolerance, and resorbability. However the collagen of animal origin, can in certain cases present resorption problems. Further, it is necessary to impregnate the meshwork with a sterile physiological serum because in the dry state, its rigidity does not permit suitable surgical use.

The present invention has the object of providing a resorbable material with a meshwork structure which is at once flexible, resistant, fluid-tight, immediately on initial use, and able to be easily sewn.

For this, the invention proposes a resorbable surgical meshwork comprised of an assembly of at least two biocompatible, resorbable threads of different natures of which one is based on cellulose and the other is a polyglycolic type synthetic material.

The assembly of these threads can be carried out by any known means such as plaiting, weaving, assembly by knitting or crochet of distinct threads.

Preferably, in accordance with the invention, it is preferred to twist together the two threads of different nature before making a woven or knitted fabric.

When such a meshwork is placed on an organ or living tissue and/or when it is placed in the presence of physiological liquids such as blood, its cellulose elements are rapidly dilated and inflated until the interstices or meshes of the meshwork are filled up in a totally hermetic manner when the percentage of cellulose is sufficient.

This impermeabilisation occurs progressively and leaves the surgeon time to position the meshwork, as the case may be enveloping an organ and providing the necessary stitches or ligatures. These operations are carried out easily and quickly thanks to the flexibility of the meshwork.

In addition to its impermeability and flexibility, such a meshwork has all the qualities useful for the application envisaged: it is well tolerated by living organisms and has sufficient mechanical strength from its elements of the polyglycolic type for supporting or making good for a certain time the wounded organs.

Finally, it shows excellent adhesiveness to living tissues.

Preferably, according to the present invention, the cellulose used is regenerated oxidized cellulose. This cellulose is known for its ability to be spun and then knitted. The preparation of regenerated oxidized cellulose is for example described in the French Patent 1071 M to which the reader is referred.

The synthetic threads used are based on polymers of glycolic acid. In an advantageous manner a copolymer of glycolic acid and lactic acid is used, preferably in the proportions of approximately 90/10 known under the mark VICRYL®.

In a typical example, a meshwork is made with a twisted VICRYL®/regenerated oxidized cellulose thread, having approximately 30% by weight of VICRYL® and 70% of cellulose.

A meshwork according to the present invention is preferably used for the surgical repair of organs or of tissues particularly vascularised such as the lungs, the bronchi, the liver or the kidneys.

It is recommended to use it on the exeresis of one or more parts of these organs, carried out particularly with the object of removing tumours.

Thus, for example, if one hepatic lobe is partially taken up by a tumour, one can, after exeresis of this latter, recover or envelop the lobe concerned by means of a meshwork according to the invention, then fix the said meshwork by means of surgical threads or other appropriate materials.

On contact with the wounded lobe and, particularly because of the presence of blood on the said lobe, the meshwork then undergoes an inflation giving it a progressive impermeabilisation. The impermeabilisation thus obtained and the initial mechanical strength of the meshwork permit this latter to efficaciously support the hepatic lobe during the time necessary for its repair. The ultimate progressive resorption of this meshwork obviates a second surgical operation.

The meshwork according to the invention can serve as a temporary prosthesis for the reinforcement or provisional replacement of diverse tissues such as cut ligaments whilst waiting for these to grow back.

Finally, it can permit the temporary fixing of a definitive prosthesis during the time necessary for its adaptation to the human or animal body.

The present invention also relates to resorbable prostheses of any form and function made totally or in part from a meshwork according to the invention.

**Claims**

1. Resorbable surgical meshwork comprising an assembly of at least two biocompatible, resorbable threads of different natures, one of which is based on regenerated oxidized cellulose, and the other one is a polyglycolic type synthetic material.

2. Surgical meshwork according to claim 1, characterised in that the meshwork comprises distinct threads of different natures.

3. Surgical meshwork according to claim 1, characterised in that the threads of different natures are twisted together.

4. Surgical meshwork according to any one of claims 1 to 3, characterised in that the synthetic threads are based on a copolymer of glycolic acid and lactic acid.

5. Surgical meshwork according to claim 4, characterised in that the synthetic threads are VICRYL® threads.

6. Surgical meshwork according to any one of claims 1 to 5 for use in the surgical repair of tissues or organs of a human or animal body.

7. Resorbable prosthesis for the temporary reinforcement of tissues of a human or animal body, made with the meshwork according to any one of claims 1 to 5.

8. Resorbable prosthesis according to claim 7 for use in the surgical repair of tissues or organs of a human or animal body.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP-A-0 159 502 (AMERICAN CYANAMID) * Claims 1-3 * | 1 | A 61 L 31/00 A 61 L 27/00 |
| A | EP-A-0 213 563 (JOHNSON & JOHNSON) * Claims 1,6 * | 1 | |

TECHNICAL FIELDS SEARCHED (Int. Cl.4)

A 61 L 31/00
A 61 L 27/00

The present search report has been drawn up for all claims

| Place of search | Date of completion of the search | Examiner |
|---|---|---|
| THE HAGUE | 09-11-1988 | PELTRE CHR. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
....................................................................
& : member of the same patent family, corresponding document

EPO FORM 1503 03.82 (P0401)